(19) Europäisches Patentamt
European Patent Office
Office européen des brevets

(11) Publication number: 0 117 100 B1

(12) **EUROPEAN PATENT SPECIFICATION**

(45) Date of publication of patent specification: **10.12.86**

(21) Application number: **84300782.4**

(22) Date of filing: **08.02.84**

(51) Int. Cl.[4]: **C 07 D 249/08,** C 07 D 403/06, A 61 K 31/41, A 01 N 43/653 // (C07D403/06, 249:08, 257:04)

(54) **Triazole antifungal agents.**

(30) Priority: **16.02.83 GB 8304282**
**28.06.83 GB 8317446**

(43) Date of publication of application:
**29.08.84 Bulletin 84/35**

(45) Publication of the grant of the patent:
**10.12.86 Bulletin 86/50**

(84) Designated Contracting States:
**AT BE CH DE FR GB IT LI LU NL SE**

(56) References cited:
**EP-A-0 040 345**
**EP-A-0 118 245**
**AT-B- 371 451**
**US-A-4 381 306**

**CHEMICAL ABSTRACTS, vol. 93, no. 21, November 24, 1980, Columbus, Ohio, USA, BUZILOVA S.R., SHUL'GINA V.M., GAREEV G.A., VERESHCHAGIN L.I. "Synthesis and properties of 2-alkyl-5-ethynyltetrazole", page 681, column 2, abstract no. 204 559u**

(73) Proprietor: **Pfizer Limited**
**Ramsgate Road**
**Sandwich Kent CT13 9NJ (GB)**
(84) **GB**

(73) Proprietor: **Pfizer Corporation**
**Calle 15 1/2 Avenida Santa Isabel**
**Colon (PA)**
(84) **BE CH DE FR IT LI LU NL SE AT**

(72) Inventor: **Richardson, Kenneth, Dr.**
**48 St. Stephens Hill**
**Canterbury Kent (GB)**
Inventor: **Whittle, Peter John, Dr.**
**5 Winchester Gardens**
**Canterbury Kent (GB)**

(74) Representative: **Moore, James William, Dr.**
**Pfizer Limited Ramsgate Road**
**Sandwich Kent CT13 9NJ (GB)**

**EP 0 117 100 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

CHEMICAL ABSTRACTS, vol. 93, no. 17, October 27, 1980, Columbus, Ohio, USA, KOTHARI, PARESH J.; STENBERG, VIRGIL I.; SINGH, SHIVA P.; PARMAR, SURENDRA S.; ZOELLNER, ROBERT W. "Carbon-13 nuclear magnetic resonance spectra of substituted-s-triazolyl tetrazoles", page 578, column 1, abstract-no. 167 085p

**Description**

This invention relates to novel triazole derivatives which have antifungal activity and are useful in the treatment of fungal infections in animals, including humans and as agricultural fungicides.

European patent application publication no. 44605 discloses certain 1,3-bis-heterocyclyl-2-aryl-propan-2-ol derivatives as fungicides and plant growth regulators. European patent application publication no. 118245 which only constitutes part of the state of the art for the purposes of Article 54(3) also discloses certain 3-fluor-1,3-bis-triazolyl-propan-2-ol antifungal agents.

According to the present invention, there are provided compounds of the formula:—

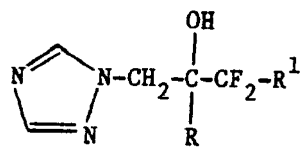

--- (I)

where R is phenyl optionally substituted by 1 to 3 substituents each independently selected from F, Cl, Br, I, $CF_3$, $C_1$—$C_4$ alkyl and $C_1$—$C_4$ alkoxy, or R is a 5-chloropyrid-2-yl group; and $R^1$ is H, Cl, phenyl optionally substituted by 1 to 3 substituents each independently selected from F, Cl, Br, I and trifluoromethyl or a 2-thienyl, 2-imidazolyl, 3-triazolyl, 5-tetrazolyl or 2- or 3-pyridyl group, each of which may optionally be substituted where appropriate by up to three substituents chosen from F, Cl, Br, I, $C_1$—$C_4$ alkyl, $C_1$—$C_4$ alkoxy or $CF_3$; and their pharmaceutically or agriculturally acceptable salts.

The invention also provides a pharmaceutical composition comprising a compound of the formula (I), or pharmaceutically acceptable salt thereof, together with a pharmaceutically acceptable diluent or carrier.

The invention further provides a compound of the formula (I), or pharmaceutically acceptable salt theeof, for use in treating fungal infections in animals, including humans.

When R is said optionally substituted phenyl group, it is preferably phenyl substituted by 1 to 3 substituents, more preferably 1 or 2 substituents, each independently selected from F, Cl, Br, I and $CF_3$. In particular in this aspect, R is 4-fluorophenyl, 4-chlorophenyl, 4-bromophenyl, 4-iodophenyl, 4-trifluoromethylphenyl, 2-chloro-4-fluorophenyl, 2,4-dichlorophenyl, 2,4-difluorophenyl, 2,4,6-trifluorophenyl or 4-bromo-2,5-difluorophenyl.

When $R^1$ is an optionally substituted 3-triazolyl, 2-imidazolyl or 5-tetrazolyl group, it is preferably a 1,2,4-triazol-3-yl; 1-methyl-1,2,4-triazol-3-yl; 2-methyl-1,2,4-triazol-3-yl; 4-methyl-1,2,4-triazol-3-yl; imidazol-2-yl or 1-methyl-imidazol-2-yl; 1-methyltetrazol-5-yl is especially preferred.

$R^1$ is preferably 4-fluorophenyl.

The compounds can be prepared by reacting an oxirane of the formula:—

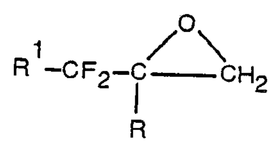

(II)

where R and $R^1$ are as defined for formula (I), with 1,2,4-triazole, preferably in the presence of a base, e.g. $K_2CO_3$. Alternatively an alkali metal salt of 1,2,4-triazole can be used, preparable e.g. from the triazole and NaH. Typically the reaction is carried out by heating the reactants together at up to 120°C in a suitable organic solvent, e.g. dimethylformamide, for up to about 24 hours. The product can then be isolated and purified conventionally.

The oxiranes (II) are obtainable conventionally, generally from the ketones of the formula:—

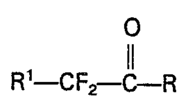

(III)

wherein R and $R^1$ are as previously defined.

This can be achieved by the reaction of (III) with dimethyloxosulphonium methylide prepared from trimethylsulphoxonium iodide and either (a) sodium hydride in dimethylsulphoxide, or (b) cetrimide (cetyltrimethylammonium bromide) and sodium hydroxide in a mixture of water and toluene or water and 1,1,1-trichloroethane. The reaction using sodium hdyride is typically carried out by stirring sodium hydride with trimethylsulphoxonum iodide at, say, room temperature. Dimethylsulphoxide (DMSO) is then added dropwise and the mixture stirred for about 30 minutes, after which time the ketone (III) is added in DMSO. The desired product is generally obtained by stirring at room temperature for about an hour. The reaction using centrimide is typically achieved by stirring the ketone (III), trimethylsulphoxonium iodide and centrimide in a mixture of 1,1,1-trichloroethane and aqueous sodium hydroxide solution for about 2 hours

at, say, 70—100°C. Whilst in either case the oxirane product (II) can be isolated if desired, it is often more convenient to convert this *in situ* to the desired product.

The ketones (III) are either known compounds or they can be prepared by conventional procedures in accordance with literature precedents. Thus, for example, the ketone of formula (III) wherein $R^1$ is H and R is 2,4-difluorophenyl can be prepared by reacting 2,4-difluorobromobenzene with n-butyllithium followed by reaction with difluoroacetic acid. Compounds wherein $R^1$ is chloro can be prepared by reacting the appropriate substituted bromobenzene with magnesium to generate the Grignard reagent which is then reacted with chlorodifluoroacetic anhydride. Alternatively, ketones of the formula (III) where $R^1$ is phenyl or substituted phenyl may be prepared by reacting the Grignard reagent with the N-methoxy-N-methylamide of the appropriate acid $R^1CF_2$—$CO_2H$, prepared according to the procedure of Nahm and Weinreb described in Tetrahedron Letters, 1981, *22*, 3815. Ketones of formula III wherein $R^1$ is a heterocyclic group are prepared from the corresponding 2-heterocyclyl-acetophenones by reacting with perchlorylfluoride. The procedures involved are well known to those skilled in the art as are the conditions for their performance and procedures for isolating, and if necessary purifying the products.

The compounds of the invention contain an optically active centre and the invention includes both the resolved and unresolved forms.

Pharmaceutically acceptable acid addition salts of the compounds of the formula (I) are those formed from strong acids which form non-toxic acid addition salts, such as hydrochloric, hydrobromic, sulphuric, oxalic and methanesulphonic acids.

The salts may be obtained by conventional procedures, e.g. by mixing solutions containing equimolar amounts of the free base and desired acid, and the required salt is collected by filtration, if insoluble, or by evaporation of the solvent.

Also included are the alkyli metal salts, preparable conventionally.

The compounds of the formula (I) and their O-esters, O-ethers and pharmaceutically acceptable salts are antifungal agents, useful in combating fungal infections in animals, including humans. For example they are useful in treating topical fungal infections in man caused by, among other organisms, species of *Candida, Trichophyton, Microsporum* or *Epidermophyton*, or in mucosal infections caused by *Candida albicans* (e.g. thrush and vaginal candidiasis). They can also be used in the treatment of systemic fungal infections caused by, for example, *Candida albicans, Crytococcus neoformans, Aspergillus fumigatus, Coccidioides, Paracoccidioides, Histoplasma* or *Blastomyces*.

The *in vitro* evaluation of the antifungal activity of the compounds can be performed by determining the minimum concentration of the test compound at which growth of the particular micro-organism in a suitable medium fails to occur. In practice, a series of agar plates, each having the test compound incorporated at a particular concentration is inoculated with a standard culture of, for example, *Candida albicans* and each plate is then incubated for 48 hours at 37°C. The plates are then examined for the presence or absence of growth of the fungus and the appropriate m.i.c. value is noted. Other microorganisms used in such tests can include *Cryptococcus neoformans, Aspergillus fumigatus, Trichophyton* spp; *Microsporum* spp; *Epidermophyton floccosum, Coccidioides immitis* and *Torulopsis glabrata.*

The *in vivo* evaluation of the compounds can be carried out at a series of dose levels by intraperitoneal or intravenous injection or by oral administration, to mice which are inoculated with a strain of *Candida albicans.* Activity is based on the survival of a treated group of mice after the death of an untreated group of mice following 48 hours observation. The dose level at which the compound provides 50% protection against the lethal effect of the infection is noted.

For human use, the antifungal compounds of the formula (I) can be administered alone, but will generally be administered in admixture with a pharmaceutical carrier selected with regard to the intended route of administration and standard pharmaceutical practice. For example, they can be administerd orally in the form of tablets containing such excipients as starch or lactose, or in capsules or ovules either alone or in admixture with excipients, or in the form of elixirs or suspensions containing flavouring or colouring agents. They can be injected parenterally, for example, intravenously, intramuscularly or subcutaneously. For parenteral administration, they are best used in the form of a sterile aqueous solution which may cotnain other substances, for example, enough salts or glucose to make the solution isotonic with blood.

For oral and parenteral administration to human patients, the daily dosage level of the antifungal compounds of the formula (I) will ba from 0.1 to 10 mg/kg (in divided doses) when administered by either the oral or parentertal route. Thus tablets or capsules of the compounds will contain from 5 mg to 0.5 g of active compound for administration singly or two or more at a time as appropriate. The physician in any event will determine the actual dosage which will be most suitable for an individual patient and it will vary with the age, weight and response of the particular patient. The above dosages are exemplary of the average case; there can, of course, be individual instances where higher or loser dosage ranges are merited, and such are within the scope of this invention.

Alternatively, the antifungal compounds of formula (I) can be administered in the form of a suppository or pessary, or they may be applied topically in the form of a lotion, solution, cream, ointment or dusting powder. For example, they can be incorporated into a cream consisting of an aqueous emulsion of polyethylene glycols or liquid paraffin; or they can be incorporated, at a concentration between 1 and 10%, into an ointment consisting of a white wax or white soft paraffin base together with such stabilizers and preservatives as may be required.

The compounds of the formula (I) and their salts also have activity against a variety of plant pathogenic fungi, including for example various rusts, mildews and moulds, and the compounds of formula I and their agriculturally acceptable salts are thus useful as agricultural fungicides for treating plants and seeds to eradicate or prevent such diseases.

The following Examples illustrate the invention:

Example 1

1,1-Difluoro-1,2-bis-(4-fluorophenyl)-3-(1H-1,2,4-triazol-1-yl)propan-2-ol

Dimethylsulphoxide (6 ml) was added under nitrogen to a mixture of sodium hydride (60% dispersion in mineral oil, 0.087 g, 2.2 mole) and trimethylsulphoxonium iodide (0.53 g, 2.4 mmole) over a period of 5 minutes, and the mixture was warmed at 45°C for a further 15 minutes. The solution was cooled and 2-(4-fluorophenyl)-2,2-4'-trifluoroacetophenone (0.53 g, 2.0 mmole) in dimethylsulphoxide (5 ml) was added over 5 minutes. The mixture was stirred for 1 hour at room temperature, water (20 ml) was then added and the mixture was extracted with toluene (3 × 25 ml). the combined toluene extracts were washed with water (2 × 20 ml), dried over $MgSO_4$ and evaporated to yield the epoxide as a colourless oil (0.53 g). 1,2,4-Triazole (0.5 g, 7.2 mmole), anhydrous potasssium carbonate (1.0 g, 7.2 mmole), and dry N,N-dimethylformamide (10 ml) were added and the mixture was heated at 50°C for 90 minutes. The solvent was then evaporated under reduced pressure and the product taken up in water (20 ml). The aqueous solution was extracted with methylene chloride (3 × 30 ml) and the combined organic extracts were washed with water (15 ml), dried over Mg $SO_4$ and evaporated to yield a colourless oil (0.67 g) which was chromatographed on silica eluting with ethyl acetate. Recrystallisation of the product from a mixture of methylene chloride and petroleum ether (b.p. 60—80°C) gave the title compound (0.3 g, 44%). m.p. 128—130°C. M/e 352 (M + 1). Found: C, 58.22; H, 3.57; N, 11.71. $C_{17}H_{13}F_4N_3O$ requires C, 58.12; H, 3.73; N, 11.96%.

Example 2

1-Chloro-2-(2,4-difluorophenyl)-1,1-difluoro-3-(1H-1,2,4-triazol-1-yl)propan-2-ol

Sodium hydride (50% suspension in mineral oil, 195 mg) was washed with a little dry diethylether under nitrogen and then dimethylsulphoxide (8 ml) and trimethylsulphoxonium iodide (850 mg) were added and the mixture warmed at 60°C until evolution of hydrogen ceased (1 hour). Dry tetrahydrofuran (8 ml) was added and the mixture cooled to −40°C. 2-Chloro-2,2,2',4'-tetrafluoroacetophenone (835 mg) was added and the mixture was stirred at −40°C for 15 minutes, allowed to warm to 10°C over 1 hour and poured onto ice (100 g). The resulting mixture was extracted with diethylether (3 × 20 ml), the ether extracts washed with brine (2 × 10 ml), dried over $MgSO_4$ and evaporated to yield the crude epoxide as a viscous oil. 1,2,4-Triazole (400 mg), anhydrous potassium carbonate (400 mg) and dry tetrahydrofuran (10 ml) were added and the mixture was heated under reflux for 18 hours. The solvent was evaporated and the residue extracted with ethyl acetate, filtered and evaporated. The product was chromatographed on silica eluting with methylene chloride containing 2% (by volume) of isopropanol and a trace of ammonium hydroxide, to give a colourless solid (210 mg) which was recrystallised from cyclohexane to give the title product as colourless crystals (0.15 g). m.p. 103—105°C. Found: C, 43.0; H, 2.7; N, 13.4. $C_{11}H_8ClF_4N_3O$ requires C, 42.7; H, 2.7; N, 13.6%.

Example 3

1,1-Difluoro-2-(2,4-difluorophenyl)-3-(1H-1,2,4-triazol-1-yl)propan-2-ol

The general procedures of Examples 1 and 2 were followed but starting with 2,2,2',4'-tetrafluoroacetophenone (1.6 g) to provide the title product (0.53 g), m.p. 122°C. Found: C, 48.1; H, 3.4; N, 15.0. $C_{11}H_9F_4N_3O$ requires C, 48.0; H, 3.3; N, 15.3%.

Example 4

1,1-Difluoro-2-(2,4-difluorophenyl)-1-(1-methyltetrazol-5-yl)-3-(1H-1,2,4-triazol-1-yl)-propan-2-ol

Dimethylsulphoxide (2 ml) was added dropwise under nitrogen to a mixture of sodium hydride (60% dispersion in mineral oil, 0.021 g, 0.52 mmole) and trimethylsulphoxonium iodide (0.125 g, 0.57 mmole). When effervescence ceased, dry tetrahydrofuran (4 ml) was added, the solution cooled to −10°C, and a solution of 2,2-difluoro-2-(1-methyl-tetrazol-5-yl)-2',4'-difluoroacetophenone (0.13 g, 0.47 mmole) in dry tetrahydrofuran (2 ml) was added. The mixture was stirred and allowed to warm gradually to 10°C over 30 minutes.

1,2,4-Triazole (0.1 g, 1.45 mmole) and anhydrous potassium carbonate (0.1 g, 0.72 mmole) were added and the mixture was heated at 70°C for 1 hour. The solvent was evaporated under reduced pressure and the product taken up in water (5 ml) and extracted with ethyl acetate (2 × 10 ml). The combined organic extracts were washed with water (3 ml), dried over magnesium sulphate and evaporated to yield the crude product as a pale yellow oil. Chromatography on silica eluting with a mixture of methylene chloride, methanol and concentrated ammonium hydroxide (93:7:1) followed by further chromatography eluting with ethyl acetate gave a white solid which was recrystallised from a mixture of ethyl acetate and petroleum ether (b.p. 60—80°C) to give the title compound (18 mg). m.p. 160—165°C. M/e 358 (M + 1). Found: C, 43.68; H, 3.14; N, 27.74. $C_{13}H_{11}N_7F_4O$ requires C, 43.70; H, 3.10; N, 27.44%.

Example 5

The following illustrate pharmaceutical compositions for the treatment of fungal infections:—

(a) *Capsule:* 71 parts by weight of the compound of Example 1 are granulated with 3 parts maize starch and 22 parts lactose and then a further 3 parts maize starch and 1 part magnesium stearate are added. The mixture is regranulated and filled into hard gelatin capsules.

(b) *Cream:* 2 parts by weight of the compound of Example 1 are dissolved in 10 parts of propylene glycol and mixed into 88 parts of a vanishing cream base.

(c) *Pessary:* 2 parts by weight of the compound of Example 2 are suspended in 98 parts of a warm liquified suppository base which is poured into moulds and allowed to solidify.

Preparation 1

2-(4-Fluorophenyl)-2,2,4'-trifluoroacetophenone

α,α-Difluoro-4-fluorophenylacetic acid (1.0 g, prepared from 4-fluorobromobenzene according to the method of Middleton and Bingham, J. Org. Chem., 1980, 45, 2883) was stirred under nitrogen with thionyl chloride (0.69 g) and dry N,N-dimethylformamide (0.036 g) at room temperature for 15 minutes and then at 70—75°C for 1 hour. The solution was cooled in ice and N,O-dimethylhydroxylamine hydrochloride (0.62 g), dry pyridine (1.5 g) and methylene chloride (15 ml) were added and the mixture stirred at 0°C for 20 minutes and at room temperature for a further 30 minutes. Water (10 ml) was added and the methylene chloride layer separated. The aqueous phase was extracted with methylene chloride (2 × 20 ml) and the combined organic extracts were dried over $MgSO_4$ and evaporated. The residue was chromatographed on silica, eluting with methylene chloride to give N-methoxy-N-methyl-α,α-difluoro-4-fluorophenyl acetamide (1.13 g).

The product was taken up in dry tetrahydrofuran (25 ml), cooled to −80°C and treated with the Grignard reagent prepared from p-fluorobromobenzene (0.9 g) and magnesium turnings (0.2 g) in dry diethylether (10 ml) with vigorous stirring under nitrogen.

The solution was allowed to warm to room temperature and allowed to stand overnight. A solution of ammonium chloride (2 g) in water (20 ml) was then added and the mixture was extracted with diethyl ether (3 × 30 ml). The combined ethereal extracts were dried over $MgSO_4$ and evaporated. The crude product was chromatographed on silica eluting with a mixture of methylene chloride and hexane (1:3) to give the title compound (0.6 g, 48%). M/e 268.

Preparation 2

2-Chloro-2,2,2',4'-tetrafluoroacetophenone

Bromo-2,4-difluorobenzene (10 g) was added to magnesium turnings (1.5 g) in dry diethyl ether (100 ml). The clear solution of the Grignard reagent was then added dropwise to a solution of chlorodifluoroacetic anhydride (10 g) in dry diethyl ether (50 ml) while stirring at −72°C under nitrogen. After 15 minutes the reaction mixture was allowed to warm to −10°C over 1 hour and then poured onto a mixture of ice (200 g) and 2N-hydrochloric acid (50 ml). The mixture was extracted with diethyl ether (3 × 100 ml), the ether extracts dried over $MgSO_4$ and evaporated to a yellow oil which was distilled to give the title product as a colourless oil (3.6 g). b.p. 50—80°C, 14 mm Hg (1860 Pa).

Preparation 3

2,2-Difluoro-2-(1-methyl-tetrazol-5-yl)-2',4'-difluoroacetophenone

*1. 2,4-Difluorobenzaldehyde*

2,4-Difluorobromobenzene (18.5 g) was added to dry diethyl ether (150 ml), cooled to −75°C and n-butyllithium (61.5 ml of a 1.55 molar solution in hexane) was added under dry nitrogen over a period of 45 minutes. The mixture was stirred at −70°C for a further 20 minutes and dry dimethylformamide (7.65 g) was added in dry diethyl ether (30 ml) at −70°C over a period of 30 minutes. The mixture was stirred for 40 minutes and allowed to warm to −50°C over a further 15 minutes. A solution of ammonium chloride (30 g) in water (100 ml) was added and the ether layer separated. The aqueous layer was further extracted with diethyl ether (2 × 150 ml) and the combined ethereal extracts were dried and evaporated to yield a pale yellow oil which was distilled at the water pump to give the desired product (13.0 g) b.p. 52—55°C at 14 mm Hg (1860 Pa).

*2. α-Trimethylsilyloxy-2,4-difluorobenzyl cyanide*

A mixture of 2,4-difluorobenzaldehyde (13.0 g) trimethylsilyl nitrile (10.0 g) and anydrous zinc iodide (0.6 g) was stirred at room temperature under nitrogen for 2 days. Further trimethylsilyl nitrile (4 g) and zinc iodide (0.2 g) were added and the mixture stirred for 18 hours when more trimethylsilyl nitrile (4 g) and zinc iodide (0.2 g) were added. The reaction mixture was stirred for a further 18 hours when the reaction was shown by NMR to be substantially complete. The mixture was distilled at the water pump to give *the product* as a pale yellow liquid (10 g, b.p. 118—119°C, 14 mm Hg (1860 Pa)).

*3. 2-(1-Methyl-tetrazol-5-yl)-2',4'-difluoroacetophenone*

N-Butyllithium (10 ml of a 1.6 molar solution in hexane) was added slowly under nitrogen to a solution of dry diisopropylamine (1.63 g) in dry tetrahydrofuran (30 ml) at −10 to −15°C. The mixture was stirred at

−10°C for 30 minutes, cooled to −75°C and a solution of the product from (2) (3.68 g) in tetrahydrofuran (20 ml) was added over 10 minutes. The mixture was stirred at −75°C for 30 minutes and a solution of 5-chloromethyl-1-methyl-tetrazole (2.0 g) in dry tetrahydrofuran (20 ml) was added over 30 minutes, the temperature being maintained between −75 and −70°C. The mixture was stirred at −75°C for a further 50 minutes, allowed to warm to room temperature over 45 minutes and stirred at room temperature for a further 30 minutes. Hydrochloric acid (36 ml 2N) was added and the mixture stirred at room temperature for 18 hours. The solution was then extracted with diethylether (2 × 100 ml), and the extracts were dried over magnesium sulphate and evaporated. Unreacted 5-chloromethyl-1-methyltetrazole was removed by distillation at 160°C and 0.4 mm Hg (53 Pa) to give a crude residue containing the desired product (1.9 g) contaminated with some 30% of unreacted 5-chloromethyl-1-methyltetrazole which was used directly for the next stage.

*4. 2,2-Difluoro-2-(1-methyl-tetrazol-5-yl)-2',4'-difluoroacetophenone*

Sodium hydride (60 mg, 60% dispersion in oil) was added at room temperature to a solution of the product from stage 3 (0.35 g) in dry tetrahydrofuran (25 ml) under dry nitrogen. When effervescence had ceased the mixture was exposed to an atmosphere of perchlorylfluoride until no more was absorbed (30 minutes). The solvent was evaporated under vacuum and the residue extracted with dry diethyl ether.

The ether solution was decanted and evaporated to give an oil. The treatment with sodium hydride and exposure to perchloroylfluoride was repeated. The product was again evaporated, extracted into diethyl ether and evaporated to yield an oil which was chromatographed on silica eluting with a mixture of hexane and ethyl acetate to yield the desired product (0.13 g). M/e 275 (M + 1).

**Claims for the Contracting States: BE CH DE FR GB IT LI LU NL SE**

1. A compound of the formula:—

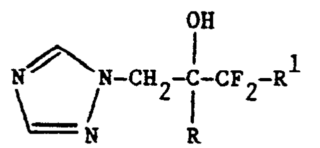

--- (I)

where R is phenyl optionally substituted by 1 to 3 substituents each independently selected from F, Cl, Br, I, $CF_3$, $C_1$—$C_4$ alkyl and $C_1$—$C_4$ alkoxy, or R is a 5-chloropyrid-2-yl group; and $R^1$ is H, Cl, phenyl optionally substituted by 1 to 3 substituents each independently selected from F, Cl, Br, I and trifluoromethyl, or a 2-thienyl, 2-imidazolyl, 3-triazolyl, 5-tetrazolyl or 2- or 3-pyridyl group, each of which may optionally be substituted where appropriate by up to three substituents chosen from F, Cl, Br, I, $C_1$—$C_4$ alkyl, $C_1$—$C_4$ alkoxy or $CF_3$; or a pharmaceutically or agriculturally acceptable salt thereof.

2. A compound or salt as claimed in claim 1 wherein R is a phenyl group substituted by 1 to 3 substituents each independently selected from F, Cl, Br, I and $CF_3$.

3. A compound or salt as claimed in claim 2, wherein R is 4-fluorophenyl, 4-chlorophenyl, 4-bromophenyl, 4-iodophenyl, 4-trifluoromethylphenyl, 2-chloro-4-fluorophenyl, 2,4-dichlorophenyl, 2,4-difluorophenyl, 2,4,6-trifluorophenyl or 4-bromo-2,5-difluorophenyl.

4. A compound or salt as claimed in any one of claims 1 to 3 wherein $R^1$ is 4-fluorophenyl.

5. 1,1-Difluoro-1,2-bis-(4-fluorophenyl)-3-(1H-1,2,4-triazol-1-yl)-propan-2-ol.

6. A compound or salt as claimed in any one of claims 1 to 3 wherein $R^1$ is 1-methyltetrazol-5-yl; 1,2,4-triazol-3-yl; 1-methyl-1,2,4-triazol-3-yl; 2-methyl-1,2,4-triazol-3-yl; 4-methyl-1,2,4-triazol-3-yl; imidazol-2-yl or 1-methyl-imidazol-2-yl.

7. 1,1-Difluoro-2-(2,4-difluorophenyl)-1-(1-methyltetrazol-5-yl)-3-(1H-1,2,4-triazol-1-yl)-propan-2-ol.

8. A pharmaceutical composition comprising a compound of the formula (I) as claimed in any one of the preceding claims, or a pharmaceutically acceptable salt thereof, together with a pharmaceutically acceptable diluent or carrier.

9. A compound of the formula (I) as claimed in any one of claims 1 to 7 or a pharmaceutically acceptable salt thereof, for use in medicine, in particular for use in treating a fungal infection in a human being.

10. A plant or seed antifungal composition, comprising a compound of the formula (I) as claimed in any one of claims 1 to 7 or an agriculturally acceptable salt thereof, togehter with an agriculturally acceptable diluent or carrier.

**Claims for the Contracting State: AT**

1. A process for preparing a compound of the formula:—

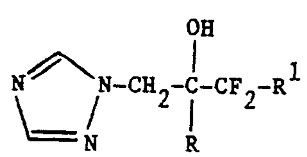

--- (I)

where R is phenyl optionally substituted by 1 to 3 substituents each independently selected from F, Cl, Br, I, $CF_3$, $C_1$—$C_4$ alkyl and $C_1$—$C_4$ alkoxy, or R is a 5-chloropyrid-2-yl group; and $R^1$ is H, Cl, phenyl optionally substituted by 1 to 3 substituents each independently selected from F, Cl, Br, I and trifluoromethyl, or a 2-thienyl, 2-imidazolyl, 3-triazolyl, 5-tetrazolyl or 2- or 3-pyridyl group, each of which may optionally be substituted where appropriate by up to three substituents chosen from F, Cl, Br, I, $C_1$—$C_4$ alkyl, $C_1$—$C_4$ alkoxy or $CF_3$; characterised by reacting 1,2,4-triazole or an alkali metal salt thereof with an oxirane of the formula:

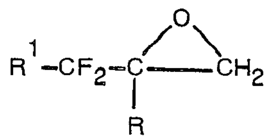

(II)

where R and $R^1$ are as defined above, and optionally forming a pharmaceutically or agriculturally acceptable salt of the product.

2. A process according to claim 1 wherein 1,2,4-triazole is reacted with the compound of formula (II) in the presence of a base.

3. A process according to claim 2 wherein the base is potassium carbonate.

4. A process according to any one of the preceding claims characterised in that it is carried out in an organic solvent at a temperature up to 120°C.

5. A process according to any one of the preceding claims characterised in that R is 4-fluorophenyl, 4-chlorophenyl, 4-bromophenyl, 4-iodophenyl, 4-trifluoromethylphenyl, 2-chloro-4-fluorophenyl, 2,4-dichlorophenyl, 2,4-difluorophenyl, 2,4,6-trifluorophenyl or 4-bromo-2,5-difluorophenyl.

6. A process according to any one of the preceding claims wherein $R^1$ is 4-fluorophenyl.

7. A process according to any one of claims 1 to 5 characterised in that $R^1$ is 1-methyltetrazol-5-yl; 1,2,4-triazol-3-yl; 1-methyl-1,2,4-triazol-3-yl; 2-methyl-1,2,4-triazol-3-yl; 4-methyl-1,2,4-triazol-3-yl; imidazol-2-yl or 1-methyl-imidazol-2-yl.

8. A process as claimed in any one of claims 1 to 5 wherein the compound of formula (I) is 1,1-difluoro-1,2-bis-(4-fluorophenyl)-3-(1H-1,2,4-triazol-1-yl)-propan-2-ol, or 1,1-difluoro-2-(2,4-difluorophenyl)-1-(1-methyltetrazol-5-yl)-3-(1H-1,2,4-triazol-1-yl)-propan-2-ol.

9. The use of a compound of the formula (I) as defined in claim 1 or an agriculturally acceptable salt thereof, as an agricultural fungicide.

**Patentansprüche für die Vertragsstaaten: BE CH DE FR GB IT LI LU NL SE**

1. Eine Verbindung der Formel

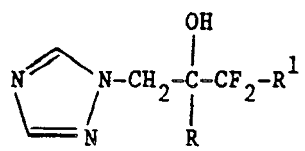

(I)

worin R Phenyl gegebenenfalls substituiert durch 1 bis 3 Substituenten jeweils unabhängig ausgewählt aus F, Cl, Br, J, $CF_3$, $C_1$—$C_4$-Alkyl und $C_1$—$C_4$-Alkoxy bedeutet oder R eine 5-Chlorpyrid-2-ylgruppe ist und $R^1$ H, Cl, Phenyl gegebenenfalls substituiert durch 1 bis 3 Substituenten jeweils unabhängig ausgewählt aus F, Cl, Br, J und Trifluormethyl oder eine 2-Thienyl-, 2-Imidazolyl-, 3-Triazolyl-, 5-Tetrazolyl- oder 2- oder 3-Pyridylgruppe darstellt, wovon jede gegebenenfalls, wenn geeignet, durch bis zu drei Substituenten jeweils ausgewählt aus F, Cl, Br, J, $C_1$—$C_4$-Alkyl, $C_1$—$C_4$-Alkoxy oder $CF_3$ substituiert sein kann, oder ein pharmazeutisch oder landwirtschaftlich annehmbares Salz hievon.

2. Verbindung oder Salz, wie in Anspruch 1 beansprucht, worin R eine Phenylgruppe substituiert durch 1 bis 3 Substituenten jeweils unabhängig ausgewählt aus F, Cl, Br, J und $CF_3$ bedeutet.

3. Verbindung oder Salz, wie in Anspruch 2 beansprucht, worin R 4-Fluorphenyl, 4-Chlorphenyl, 4-Bromphenyl, 4-Jodphenyl, 4-Trifluormethylphenyl, 2-Chlor-4-fluorphenyl, 2,4-Dichlorphenyl, 2,4-Difluorphenyl, 2,4,6-Trifluorphenyl oder 4-Brom-2,5-difluorphenyl ist.

4. Verbindung, wie in einem der Ansprüche 1 bis 3 beansprucht, worin $R^1$ 4-Fluorphenyl ist.

5. 1,1-Difluor-1,2-bis-(4-fluorphenyl)-3-(1H-1,2,4-triazol-1-yl)-propan-2-ol.

6. Verbindung, wie in einem der Ansprüche 1 bis 3 beansprucht worin $R^1$ 1-Methyltetrazol-5-yl, 1,2,4-Triazol-3-yl, 1-Methyl-1,2,4-triazol-3-yl, 2-Methyl-1,2,4-triazol-3-yl, 4-Methyl-1,2,4-triazol-3-yl, Imidazol-2-yl oder 1-Methylimidazol-2-yl ist.

7. 1,1-Difluor-2-(2,4-difluorphenyl)-1-(1-methyltetrazol-5-yl)-3-(1H-1,2,4-triazol-1-yl)-propan-2-ol.

8. Pharmazeutische Zusammensetzung umfassend eine Verbindung der Formel (I), wie in einem der vorhergehenden Ansprüche beansprucht, oder ein pharmazeutisch annehmbares Salz hievon zusammen mit einem pharmazeutisch annehmbaren Verdünnungsmittel oder Träger.

9. Verbindung der Formel (I), wie in einem der Ansprüche 1 bis 7 beansprucht, oder ein pharmazeutisch annehmbares Salz hievon zur Verwendung in der Medizin, insbesondere zur Verwendung beim Behandeln einer Pilzerkrankung bei einem Menschen.

10. Pflanzen- oder Samenantipilzzusammensetzung umfassend eine Verbindung der Formel (I), wie in einem der Ansprüche 1 bis 7 beansprucht, oder ein landwirtschaftlich annehmbares Salz hievon zusammen mit einem landwirtschaftlich annehmbaren Verdünnungsmittel oder Träger.

**Patentansprüche für den Vertragsstaat: AT**

1. Verfahren zum Herstellen einer Verbindung der Formel

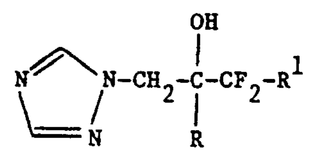

--- (I)

worin R Phenyl gegebenenfalls substituiert durch 1 bis 3 Substituenten jeweils unabhängig ausgewählt aus F, Cl, Br, J, $CF_3$, $C_1$—$C_4$-Alkyl und $C_1$—$C_4$-Alkoxy bedeutet oder R eine 5-Chlorpyrid-2-ylgruppe ist und $R^1$ H, Cl, Phenyl gegebenenfalls substituiert durch 1 bis 3 Substituenten jeweils unabhängig ausgewählt aus F, Cl, Br, J und Trifluoromethyl oder eine 2-Thienyl-, 2-Imidazolyl-, 3-Triazolyl-, 5-Tetrazolyl- oder 2- oder 3-Pyridylgruppe darstellt, wovon jede gegebenenfalls, wenn geeignet, durch bis zu drei Substituenten jeweils ausgewählt aus F, Cl, Br, J, $C_1$—$C_4$-Alkyl, $C_1$—$C_4$-Alkoxy oder $CF_3$ substituiert sein kann, gekennzeichnet durch Umsetzen von 1,2,4-Triazol oder eines alkalimetallsalzes hievon mit einem Oxiran der Formel

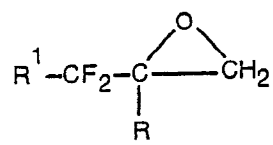

(II)

worin R und $R^1$ wie oben definiert sind, und gegebenenfalls Bilden eines pharmazeutisch oder landwirtschaftlich annehmbaren Salzes des Produktes.

2. Verfahren nach Anspruch 1, worin 1,2,4-Triazol mit der Verbinding der Formel (II) in Anwesenheit einer Base umgesetzt wird.

3. Verfahren nach Anspruch 2, worin die Base Kaliumcarbonat ist.

4. Verfahren nach einem der vorhergehenden Ansprüche, dadurch gekennzeichnet, daß es in einem organischen Lösungsmittel bei einer Temperatur bis zu 120°C durchgeführt wird.

5. Verfahren nach einem der vorhergehenden Ansprüche, dadurch gekennzeichnet, daß R 4-Fluorphenyl, 4-Chlorphenyl, 4-Bromphenyl, 4-Jodphenyl, 4-Trifluormethylphenyl, 2-Chlor-4-fluorphenyl, 2,4-Dichlorphenyl, 2,4-Difluorphenyl, 2,4,6-Trifluorphenyl oder 4-Brom-2,5-difluorphenyl ist.

6. Verfahren nach einem der vorhergehenden Ansprüche, worin $R^1$ 4-Fluorphenyl ist.

7. Verfahren nach einem der Ansprüche 1 bis 5, dadurch gekennzeichnet, daß $R^1$ 1-Methyltetrazol-5-yl, 1,2,4-Triazol-3-yl, 1-Methyl-1,2,4-triazol-3-yl, 2-Methyl-1,2,4-triazol-3-yl, 4-Methyl-1,2,4-triazol-3-yl, Imidazol-2-yl oder 1-Methylimidazol-2-yl ist.

8. Verfahren, wie in einem der Ansprüche 1 bis 5 beansprucht, worin die Verbindung der Formel (I) 1,1-Difluor-1,2-bis-(4-fluorphenyl;-3-(1H-1,2,4-triazol-1-yl)-propan-2-ol oder 1,1-Difluor-2-(2,4-difluorphenyl)-1-(1-methyltetrazol-5-yl)-3-(1H-1,2,4-triazol-1-yl)-propan-2-ol ist.

9. Verwendung einer Verbindung der Formel (I), wie in Anspruch 1 definiert, oder eines landwirtschaftlicht annehmbaren Salzes hievon als landwirtschaftliches Fungizid.

**Revendications pour les Etats Contractants: BE CH DE FR GB IT LI LU NL SE**

1. Composé de formule:

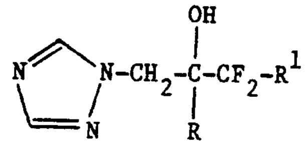

--- (I)

dans laquelle R est un radical phényle, éventuellement substitué par de 1 à 3 substituants dont chacun est indépendament choisi entre F, Cl, Br, I, $CF_3$, alkyle en $C_1$—$C_4$ et alcoxy en $C_1$—$C_4$, ou R est un groupe 5-chloropyrid-2-yle; et $R_1$ représente H, Cl, phényle éventuellement substitué par de 1 à 3 substituants dont chacun est choisi indépendamment entre F, Cl, Br, I et trifluorométhyle, ou un radical 2-thiényle, 2-imidazolyle, 3-triazolyle, 5-tétrazolyle ou 2- ou 3-pyridyle, chacun de ces radicaux pouvant être

éventuellement substitué aux sommets appropriés par jusqu'à 3 substituants choisis parmi F, Cl, Br, I, alkyle en $C_1$—$C_4$, alcoxy en $C_1$—$C_4$, ou $CF_3$; ou un sel acceptable sur le plan pharmaceutique ou agricole.

2. Composé ou sel selon la revendication 1, dans lequel R est un groupe phényle substitué par de 1 à 3 substituants dont chacun est indépendamment choisi entre F, Cl, Br, I et $CF_3$.

3. Composé ou sel selon la revendication 2, dans lequel R est un radical 3-fluorophényle, 4-chlorophényle, 4-bromophényle, 4-iodophényle, 4-trifluorométhylphényle, 2-chloro-4-fluorophényle, 2,4-dichlorophényle, 2,4-difluorophényle, 2,4,6-trifluorophényle ou 4-bromo-2,5-difluorophényle.

4. Composé selon l'une quelconque des revendications 1 à 3, dans lequel $R^1$ est un radical 4-fluorophényle.

5. 1,1-difluoro-1,2-bis(4-fluorophényl)-3-(1H-1,2,4-triazol-1-yl)-propane-2-ol.

6. Composé selon l'une quelconque des revendications 1 à 3 dans lequel $R^1$ est un radical 1-méthyltétrazol-5-yle) 1,2,4-triazol-3-yle; 1-methyl-1,2,4-triazol-3-yle; 2-méthyl-1,2,4-triazol-3-yle; 4-méthyl-1,2,4-triazol-3-yle; imidazol-2-yle ou 1-méthyl-imidazol-2-yle.

7. 1,1-difluoro-2-(2,4-difluorophényl)-1-(1-méthyltétrazol-5-yl)-3-(1H-1,2,4-triazol-1-yl)-propane-2-ol.

8. Composition pharmaceutique renfermant un composé de formule (I) tel que revendiqué dans l'une quelconque des revendications précédentes, ou un sel pharmaceutiquement acceptable d'un tel composé avec un diluant ou un support pharmaceutiquement acceptable.

9. Composé de formule (I) selon l'une quelconque des revendications 1 à 7 ou un sel pharmaceutiquement acceptable d'un tel composé, pour son usage en médecine, en particulier pour traiter une infection fongique chez l'être humain.

10. Composition anti-fongique pour plantes ou semences, comprenant un composé de formule (I) tel que revendiqué dans l'une quelconque des revendications 1 à 7 ou un sel de ce composé acceptable sur le plan agricole, avec un diluant ou un support acceptable sur le plan agricole.

**Revendications pour l'Etat Contractant: AT**

1. Procédé de préparation d'un composé de formule:

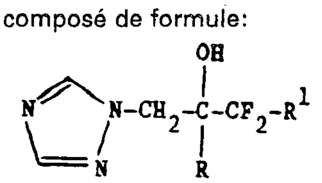

--- (I)

dans laquelle R est un radical phényle, éventuellement substitué par de 1 à 3 substituants dont chacun est indépendament choisi entre F, Cl, Br, I, $CF_3$, alkyle en $C_1$—$C_4$ et alcoxy en $C_1$—$C_4$, ou R est un groupe 5-chloropyrid-2-yle; et $R_1$ représente H, Cl, phényle éventuellement substitué par de 1 à 3 substituants dont chacun est choisi indépendamment entre F, Cl, Br, I et trifluorométhyle, ou un radical 2-thiényle, 2-imidazolyle, 3-triazolyle, 5-tétrazolyle ou 2- ou 3-pyridyle, chacun de ces radicaux pouvant être éventuellement substitué aux sommets appropriés par jusqu'à 3 substituants choisis parmi F, Cl, Br, I, alkyle en $C_1$—$C_4$, alcoxy en $C_1$—$C_4$, ou $CF_3$; caractérisé en ce qu'il consiste à faire réagir le 1,2,4-triazole ou un sel de métal alcalin de ce composé avec un oxirane de formule:

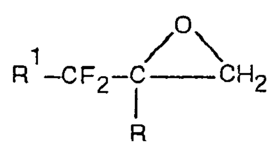

(II)

où R et $R^1$ sont tels que définis ci-dessus, et éventuellement à former un sel acceptable sur le plan pharmaceutique ou agricole de ce composé.

2. Procédé selon la revendication 1, dans lequel on fait réagir le 1,2,4-triazole avec le composé de formule (II) en présence d'une base.

3. Procédé selon la revendication 2, dans lequel la base est le carbonate de potassium.

4. Procédé selon l'une quelconque des revendications précédentes, caractérisé en ce qu'il est mis en oeuvre dans un solvant organique à une température pouvant atteindre 120°C.

5. Procédé selon l'une quelconque des revendications précédentes, dans lequel R est un radical 4-fluorophényle, 4-chlorophényle, 4-bromophényle, 4-iodophényl, 4-trifluorométhylphényle, 2-chloro-4-fluorophényle, 2,4-dichlorophényle, 2,4-difluorophényle, 2,4,6-trifluorophényle ou 4-bromo-2,5-difluorophényle.

6. Procédé selon l'une quelconque des revendications précédentes, dans lequel $R^1$ est un radical 4-fluorophényle.

7. Procédé selon l'une quelconque des revendications 1 à 5 dans lequel $R^1$ est un radical 1-méthyltétrazol-5-yle; 1,2,4-triazol-3-yle; 1-méthyl-1,2,4-triazol-3-yle; 2-méthyl-1,2,4-triazol-3-yle; 4-méthyl-1,2,4-triazol-3-yle; imidazol-2-yle ou 1-méthyl-imidazol-2-yle.

8. Procédé selon l'une quelconque des revendications 1 à 5, dans lequel le composé de formule (I) est le 1,1-difluoro-1,2-bis-(4-fluorophényl)-3-(1H-1,2,4-triazol-1-yl)-propane-2-ol ou le 1,1-difluoro-2-(2,4-difluorophényl)-1-(1-méthyltétrazol-5-yl)-3-(1H-1,2,4-triazol-1-yl)-propane-2-ol.

9. Utilisation, comme fongicide agricole, d'un composé de formule (I) tel que défini dans la revendication 1 ou d'un sel acceptable sur le plan agricole d'un tel composé.